(19) Europäisches Patentamt / European Patent Office / Office européen des brevets

(11) **EP 2 764 931 A1**

(12) **EUROPEAN PATENT APPLICATION**
published in accordance with Art. 153(4) EPC

(43) Date of publication:
**13.08.2014 Bulletin 2014/33**

(21) Application number: **12838555.6**

(22) Date of filing: **04.10.2012**

(51) Int Cl.:
**B09B 3/00** (2006.01)   **C05F 1/00** (2006.01)
**C05F 3/00** (2006.01)   **C05F 5/00** (2006.01)
**C05F 9/00** (2006.01)   **C05G 5/00** (2006.01)
**C12M 1/00** (2006.01)   **C12N 1/00** (2006.01)
**C12N 1/20** (2006.01)   **C12N 11/08** (2006.01)

(86) International application number:
**PCT/JP2012/075791**

(87) International publication number:
**WO 2013/051648 (11.04.2013 Gazette 2013/15)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.10.2011 JP 2011223230**

(71) Applicants:
• **Iga Bio Research Co., Ltd.**
  **Sakai-shi, Osaka 590-0132 (JP)**
• **Kuraray Co., Ltd.**
  **Kurashiki-shi, Okayama 710-0801 (JP)**

(72) Inventors:
• **SAKAI, Takuo**
  **Osaka 590-0132 (JP)**
• **HASHIMOTO, Tamotsu**
  **Tokyo 100-8115 (JP)**
• **YOSHIHARA, Mototsugu**
  **Ehime 793-8585 (JP)**

(74) Representative: **Müller-Boré & Partner
Patentanwälte PartG mbB
Friedenheimer Brücke 21
80639 München (DE)**

(54) **METHOD AND APPARATUS FOR DECOMPOSING BIOLOGICAL SUBSTANCE**

(57)   A method for decomposing a biological material by microorganisms supported on a carrier, characterized in that the carrier is an aqueous polyvinyl alcohol carrier and 20 to 500 parts by weight of the biological material and 5 to 70 parts by weight of water based on 100 parts by weight of the aqueous polyvinyl alcohol carrier are mixed and agitated in a reaction vessel. The method enables the efficient decomposition of a biomass. The method also enables the efficient production of a liquid fertilizer comprising an aqueous solution containing a decomposition product of a biomass.

[FIG. 1]

EP 2 764 931 A1

**Description**

TECHNICAL FIELD

The present invention relates to a method for decomposing a biological material by microorganisms supported on a carrier and a decomposition apparatus. The present invention also relates to a method for producing a liquid fertilizer by decomposing a biological material by microorganisms supported on a carrier.

BACKGROUND ART

In twentieth century, human-beings have constructed modern civilization based on the heavy use of non-renewable fossil resources. However, as human life has been normalized, such a life style has been a factor of global environmental deterioration due to byproducts, along with problems such as runup of a resource cost and tight resource supply. Thus, there has been strongly needed developing technique capable of efficiently utilizing recyclable resources.

In particular, biological materials (hereinafter, referred to as "biomass") are the largest recyclable resource in the earth, and efficient recycling of such materials is desired. To date, technique for processing a biomass such as domestic garbage (hereinafter, simply referred to as "garbage"), food processing waste and agricultural waste is based on a so-called indole method for producing a fertilizer by partial decomposition of a biomass utilizing microorganism activity. For this purpose, many apparatuses have been developed.

However, in a conventional method for fermentatively decomposing a solid biomass by adding microorganisms (solid-phase fermentation), the amount of the microorganisms is smaller than volume of the biomass as a raw material. The method, therefore, has a problem that in the initial decomposition stage, decomposition by resident microorganisms carried by the biomass itself is predominant rather than decomposition by the microorganisms added to the biomass. It often leads to off-odor (smell) production and spoiling during a fermentation process. There have been, therefore, attempts for efficiently decomposing a biomass by solid-state fermentation; for example, fermentation is effected using microorganisms having special properties (for example, thermophilic bacteria) while inhibiting activity of resident microorganisms.

None of these methods can, however, completely decompose a biomass to a liquid product. It is thus difficult to establish a high-density microorganism environment suitable for such decomposition. In this context, there have been proposed an apparatus where in addition to microorganisms, a filler is added to a processing tank for decomposition, and a material sustainingly releasing microorganisms in which microorganisms are fixed on a substrate via a binder.

Patent Reference 1 has disclosed an apparatus for processing garbage wherein a garbage and a filler are blended and the garbage is decomposed by activity of the microorganisms in a processing tank. The reference describes the use of foamed resin pellets as a filler. It describes that the foamed resin is suitably selected from foaming products of a synthetic resin such as polystyrene, polyethylene, polypropylene and polyurethane. The style of the foamed material can be of closed-cell foaming or open-cell foaming, and the reference describes that closed-cell foaming is preferable since pieces of a garbage do not enter the foam and it facilitates contact of the garbage with water and the air through a superficial skin layer. Thus, according to the description, the filler itself is unchanged as the garbage is decomposed, and mixing the filler with the garbage allows for smooth contact of the garbage with water and the air, which promotes decomposition. It, however, does not seem that its decomposition ability is fully satisfactory.

PRIOR ART REFERENCE

PATENT REFERENCE

Patent Reference 1: JP08-112582 A

SUMMARY OF THE INVENTION

PROBLEM TO BE SOLVED BY THE INVENTION

To solve the above problems, an objective of the present invention is to provide a method for decomposing a biomass, allowing for efficiently decomposition of a biomass.

MEANS FOR SOLVING PROBLEM

The above problems can be solved by providing a method for decomposing a biological material by microorganisms supported on a carrier, characterized in that the carrier is an aqueous polyvinyl alcohol carrier and 20 to 500 parts by weight of the biological material and 5 to 70 parts by weight of water based on 100 parts by weight of the aqueous polyvinyl alcohol carrier are mixed and agitated in a reaction vessel.

Here, preferably, an aqueous solution containing a decomposition product is taken out of the reaction vessel. Also preferably, an aqueous solution containing a decomposition product is continuously taken out of the reaction vessel while water is continuously or intermittently supplied to the reaction vessel.

The microorganisms are prefer ably bacteria of Bacillus genus. Preferably, the biological material is at least one selected from the group consisting of an agricultural waste, a livestock waste, a fishery waste, a food processing waste and a kitchen waste. Also preferably, 20 to 300 parts by weight of a hard carrier based on 100 parts by weight of the aqueous polyvinyl alcohol carrier is further added to the reaction vessel and the mixture is then mixed and agitated.

The above problems can be also solved by providing a method for producing a liquid fertilizer by decomposing a biological material by microorganisms supported on a carrier, characterized in that the carrier is an aqueous polyvinyl alcohol carrier, and 20 to 500 parts by weight of the biological material and 5 to 70 parts by weight of water based on 100 parts by weight of the aqueous polyvinyl alcohol carrier are mixed and agitated, and an aqueous solution containing a decomposition product is taken out of the reaction vessel.

The above problems can be also solved by providing an apparatus for decomposing a biological material by microorganisms supported on a carrier, comprising a reaction vessel which contains an aqueous polyvinyl alcohol carrier supporting microorganisms; means for supplying water to the reaction vessel; means for agitating a biological material in the reaction vessel; and means for taking an aqueous solution containing a decomposition product out of the reaction vessel.

EFFECT OF THE INVENTION

According to a decomposition method of the present invention, a biomass can be efficiently decomposed. Furthermore, a liquid fertilizer as an aqueous solution containing a decomposition product of a biomass can be efficiently produced.

BRIEF DESCRIPTION OF THE DRAWINGS

FIG. 1 shows an example of a decomposition apparatus of the present invention.
FIG. 2 shows germination of Japanese mustard spinach seeds in Example 5.
FIG. 3 shows development of Japanese mustard spinach in Example 6.

MODE FOR CARRYING OUT THE INVENTION

In the present invention, it is important to use an aqueous polyvinyl alcohol carrier as a carrier supporting microorganisms. Polyvinyl alcohol has many hydroxy groups which endow it with hydrophilicity and biocompatibility, and therefore, it is suitable as a microorganism carrier. An aqueous polyvinyl alcohol carrier is preferably polyvinyl alcohol hydrous gel, more preferably acetalized polyvinyl alcohol hydrous gel. It allows for increase of retained microorganisms and ensuring durability to repetitive use. Furthermore, an aqueous polyvinyl alcohol carrier used in the present invention is very water-retentive. Therefore, even if the carrier is deformed by an external force, water is not easily released so that an environment suitable for microorganism inhabitation can be maintained. On the other hand, if a closed-cell foamof a synthetic resin such as polystyrene, polyethylene, polypropylene and polyurethane is used as a carrier supporting microorganisms, foams are separate, that is, not continuous, so that water or the air is not penetrative, and therefore, it is not suitable as a carrier supportingmicroorganisms. If a continuous foam of a synthetic resin is used as a carrier supporting microorganisms, deformation by an external force easily leads to release of water in contrast to an aqueous polyvinyl alcohol carrier used in the present invention and thus an environment suitable for microorganism inhabitation cannot be maintained, resulting in poor microorganism inhabitation.

It is particularly preferable to use a spherical hydrous gel prepared by adding dropwise an aqueous solution of polyvinyl alcohol and a water-soluble macromolecular polysaccharide such as an alginate to an aqueous solution containing polyvalent metal ions such as an aqueous solution of calcium chloride to form spheres and then acetalizing the spheres using, for example, formalin. Since a uniform spherical gel can be prepared as described above, a contact efficiency of the surface of the spherical gel carrier carrying microorganisms with a biomass is increased, so that not only a decomposition efficiency but also durability during repetitive use can be improved.

Polyvinyl alcohol used for an aqueous polyvinyl alcohol carrier of the present invention is prepared by polymerizing a vinyl carboxylate such as vinyl acetate and then saponifying the polymer produced. In the light of strength, water resistance and the like, the polyvinyl alcohol preferably has an average polymerization degree of 1000 or more, particularly preferably 1500 or more. Furthermore, a saponification degree is preferably 95 mol% or more, more preferably 98 mol% or more, particularly preferably 99.8 mol% in the light of water resistance and the like. Although an unmodified polyvinyl alcohol is generally used in the present invention, a variety of modified polyvinyl alcohols may be used as long as the effects of the present invention are not deteriorated.

An average polymerization degree as described above is calculated by the following equation.

$$\text{Average polymerization degree} = ([\eta] \times 10^3 / 7.94)^{(1/0.62)}$$

In the equation, $[\eta]$ represents a limiting viscosity, which was measured as follows. A polyvinyl alcohol polymer was completely saponified and then reacetified, and a limiting viscosity $[\eta]$ of the resulting polymer was measured in acetone at 30 °C using an Ubbelohde type viscometer.

An aqueous polyvinyl alcohol carrier used in the present invention preferably has a three-dimensional network structure having continuous pores. Such a structure dramatically can increase the surface area of an aqueous polyvinyl alcohol carrier, leading to significant improvement in microorganism retention and an efficiency of contact with a biomass and therefore, improvement in an efficiency of biomass decomposition. A water content of an aqueous polyvinyl alcohol carrier is suitably 50 to 95 % by weight. If a water content is less than 50 % by weight, the amount of retained microorganisms may be insufficient, and it is, therefore, more suitably 70 % by weight or more. If a water content is more than 95 % by weight, carrier strength may be reduced, and it is, therefore, more suitably 92 % by weight or less.

A sphere-equivalent diameter of an aqueous polyvinyl alcohol carrier is preferably 0.5 to 6 mm. With a carrier size within the range, a surface area per unit weight of the carrier can be increased while carrier processability is improved. Thus, microorganism retention and an efficiency of contact with a biomass can be further increased, leading to improvement in an efficiency of biomass decomposition.

There are no particular restrictions to microorganisms supported on an aqueous polyvinyl alcohol carrier in the present invention as long as they can decompose a biomass, and they are preferably bacteria of Bacillus genus. Examples of bacteria of Bacillus genus include Bacillus amyloliquefaciens IFO14141, Bacillus circulans IFO3329, Bacillus coagulans IFO12583, Bacillus licheniformis IFO12195, Bacillus megaterium IFO AKU212, Bacillus subtilis IFO3134 and Bacillus subtilis TS-A (FERM P-18351). Among these, microorganisms supported on a carrier in the present invention is more preferably Bacillus natto (Bacillus subtilis) and/or congenetic bacteria. The carrier can carry bacteria of one Bacillus genus or two or more Bacillus genera.

In the present invention, microorganisms supported on an aqueous polyvinyl alcohol carrier can be obtained by conventional culturing. The culturing conditions depends on the type of microorganisms and can be appropriately selected for achieving the maximum amount of the microorganisms. A culture medium for microorganisms contains naturally-occurring organic materials such as starch, peptone, casein hydrolysate, dextrin, yeast extract and dextrose. An inorganic salt such as a phosphate and a potassium salt can be optionally added to a culture medium in a proper amount. Furthermore, a nutritional source such as wheat bran, rice bran, soybean flour and rice hull can be optionally added to a culture medium in a proper amount. There are also no particular restrictions to the culturing conditions, and the conditions can be appropriately selected from those suitable for common microorganism growth such as aerated liquid culture and solid culture.

There are no particular restrictions to a method for preparing an aqueous polyvinyl alcohol carrier supporting microorganisms, and it can be prepared by, for example, a method described below.

An dispersion of an aqueous polyvinyl alcohol carrier in water is prepared. To the dispersion, a culture medium made of naturally-occurring organic materials and so on is added and the mixture is well mixed. Here, it is preferable to add 1 to 5 parts by weight of the culture medium to 100 parts by weight of the aqueous polyvinyl alcohol carrier. Microorganisms are added to the mixture, which is then well mixed to grow microorganisms, allowing for supporting microorganisms by the carrier. The conditions for culturing microorganisms are, but not limited to, 20 to 30 °C and 10 to 40 hours when the microorganisms are bacteria of Bacillus genus. The above temperature range is preferable because a growth rate of culturing can be maintained for a long time. The above time range is preferable because an adequate amount of microorganisms for decomposing a biomass can be supported on a carrier. Under such culture conditions, the Bacillus genus grows to 10 to 100 folds of the original amount. It is preferable to stir a culture medium containing the carrier at intervals of 4 to 8 hours. Thus, the microorganisms can be efficiently supported on the aqueous polyvinyl alcohol carrier.

Particularly in the light of a simpler production process, it is also preferable to support microorganisms on an aqueous polyvinyl alcohol carrier by preliminarily dissolving a culture medium in water, blending the medium solution with an aqueous polyvinyl alcohol carrier and adding microorganisms to the mixture. Although there are no particular restrictions to the conditions for culturing microorganisms, microorganisms are preferably cultured at a temperature of room temperature to 28 °C for Bacillus genus. Alternatively, microorganisms can be supported on an aqueous polyvinyl alcohol carrier by adding microorganisms to a culture medium containing naturally-occurring organic materials for culturing and then adding the aqueous polyvinyl alcohol carrier to the mixture. It is preferable to stir a culture medium containing the carrier at intervals of 4 to 8 hours. Thus, the microorganisms can be efficiently supported on the aqueous polyvinyl alcohol carrier.

There are no particular restrictions to a biological material to be decomposed in the present invention as long as it can be decomposed by microorganisms, and preferably the material is at least one waste selected from the group consisting of agricultural waste, livestock waste, fishery waste, food processing waste and kitchen waste. Among these, a suitable biological material to be decomposed in the present invention is kitchen waste, particularly domestic garbage.

For efficient decomposition of a biological material, the material is more preferably one of the above wastes, further preferably a waste consisting of a single plant or animal species. Here, turfgrass can be an example of a biological material as a single plant species. In general, turfgrass must be periodically manicured in a turfy area such as a golf course, a soccer ground and a schoolyard. If the turfgrass clipplings are left, they may adversely affect growth of turfgrass. Furthermore, there is a problem that disposal of the turfgrass clipplings costs time and money. Thus, the turfgrass clipplings can be efficiently treated without time or cost by decomposing the turfgrass by a decomposition method of the present invention. Furthermore, an aqueous solution containing a decomposition product obtained by decomposition of turfgrass can be used as a liquid fertilizer. In other words, turfgrass clipplings can be regenerated as a fertilizer for turfgrass growing, leading to construction of a recycling system.

In a decomposition method of the present invention, an aqueous polyvinyl alcohol carrier supporting microorganisms, biological material and water are mixed in a reaction vessel and agitated. Here, the amount of a biological material added is 20 to 500 parts by weight based on 100 parts by weight of an aqueous polyvinyl alcohol carrier. If the amount of a biological material in a reaction vessel is less than 20 parts by weight based on 100 parts by weight of an aqueous polyvinyl alcohol carrier, the amount of microorganisms is too large in relation to a biological material, which is inefficient. The amount of a biological material in a reaction vessel is preferably 30 parts by weight or more, more preferably 50 parts by weight or more. If the amount of a biological material in a reaction vessel is more than 500 parts by weight based on 100 parts by weight of an aqueous polyvinyl alcohol carrier, the amount of microorganisms may be too small in relation to the amount of the biological material, leading to inefficient decomposition. The amount of a biological material in a reaction vessel is preferably 300 parts by weight or less, more preferably 200 parts by weight or less based on 100 parts by weight of an aqueous polyvinyl alcohol carrier.

The amount of water added is 5 to 70 parts by weight based on 100 parts by weight of an aqueous polyvinyl alcohol carrier supporting microorganisms. With the amount of water within this range, a biomass can be efficiently decomposed. The amount of water in a reaction vessel is preferably 10 parts by weight or more, more preferably 20 parts by weight or more based on 100 parts by weight of an aqueous polyvinyl alcohol carrier.

In a reaction vessel, in addition to an aqueous polyvinyl alcohol carrier supporting microorganisms, a biological material and water, 20 to 300 parts by weight of a hard carrier can be further added based on 100 parts by weight of the aqueous polyvinyl alcohol carrier before mixing and agitation. A material of the hard carrier is preferably a material harder than an aqueous polyvinyl alcohol carrier such as a hard resin, a ceramic, a metal piece and a wood chip. Agitation of a biological material and a hard carrier in a reaction vessel causes crush of a biological material by a hard carrier, accelerating decomposition of a biological material. If the amount of a hard carrier in a reaction vessel is less than 20 parts by weight based on 100 parts by weight of an aqueous polyvinyl alcohol carrier, a crushing force is insufficient. The amount of a hard carrier in a reaction vessel is preferably 30 parts by weight or more, more preferably 50 parts by weight or more. If the amount of a hard carrier in a reaction vessel is more than 300 parts by weight based on 100 parts by weight of an aqueous polyvinyl alcohol carrier, the hard carrier is too much, leading to an insufficient decomposition efficiency. The amount of a hard carrier in a reaction vessel is preferably 200 parts by weight or more, more preferably 250 parts by weight or more. A hard carrier preferably has a sphere-equivalent diameter of 0.5 to 15 mm.

Then, an aqueous polyvinyl alcohol carrier supporting microorganisms with the above proportion, a biological material and water are mixed and agitated in a reaction vessel, to initiate decomposition of a biological material by the microorganisms supported on a carrier. There are no particular restrictions to a mixing method or an agitation method, and an aqueous polyvinyl alcohol carrier, a biological material and water can be placed in a reaction vessel with a predetermined size, and the content in the reaction vessel can be then agitated using a known agitating means.

In a decomposition method of the present invention, it is preferable to take an aqueous solution containing a decomposition product out of the reaction vessel. Specifically, a microorganism-supporting carrier, a biological material and water are mixed in a reaction vessel, and after a predetermined time or after decomposition of the biological material to a predetermined level, an aqueous solution containing a decomposition product is taken out of a reaction vessel. Thus, an aqueous solution containing a decomposition product does not remain in the reaction vessel.

It is preferable to continuously take an aqueous solution containing a decomposition product out of a reaction vessel while continuously or intermittently supplying water to the reaction vessel. Thus, the amount of water in the reaction vessel can be adjusted to 5 to 70 parts by weight based on 100 parts by weight of a microorganism-supporting carrier. Furthermore, decomposition can be continuously conducted by successively adding the biological material to the reaction vessel as the material is decreased by decomposition.

In the present invention, a liquid fertilizer can be efficiently produced by decomposing a biological material by microorganisms supported on a carrier and taking an aqueous solution containing a decomposition product out of a reaction vessel.

A decomposition apparatus of the present invention has a reaction vessel which contains an aqueous polyvinyl alcohol carrier supporting microorganisms; means for supplying water to the reaction vessel; means for agitating a biological material in the reaction vessel; and means for taking an aqueous solution containing a decomposition product out of the reaction vessel.

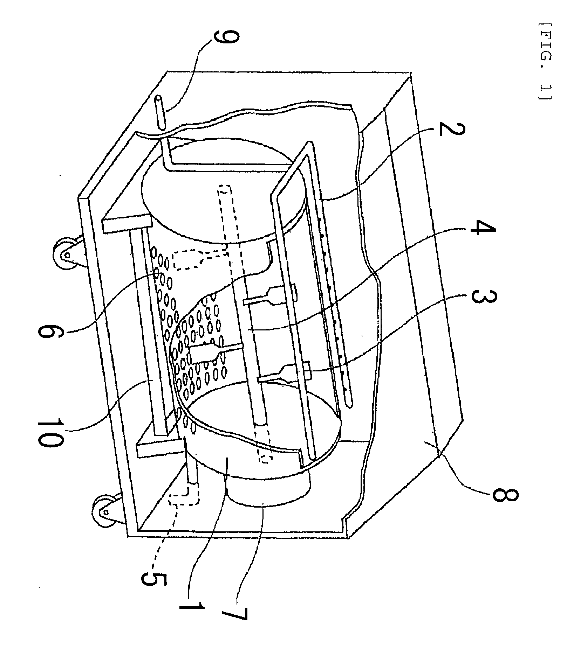

FIG. 1 shows an example of a decomposition apparatus of the present invention. This decomposition apparatus has a reaction vessel 1, a water spray pipe 2 for supplying water to the reaction vessel 1, an agitation scraper 3 for agitating a content in the reaction vessel 1 and a shaft 4 equipped with the agitation scraper, and a pipe for removing an aqueous solution 5 which removes an aqueous solution containing a decomposition product.

An aqueous polyvinyl alcohol carrier supporting microorganisms is placed in the reaction vessel 1 in the decomposition apparatus. Here, the aqueous polyvinyl alcohol carrier in the reaction vessel 1 is preferably the microorganism-supporting carrier described above.

The water spray pipe 2 is a pipe for supplying water to the reaction vessel 1. There are no particular restrictions to a method for supplying water, and preferably, water is sprayed over the reaction vessel 1 from the water spray pipe with pores aligned at proper intervals which is disposed over the reaction vessel, as shown in FIG. 1. There are also no particular restrictions to the number of the water spray pipes 2, the size of the pores, the number of the pores and the like, and they can be appropriately chosen such that water is supplied to the whole content in the reaction vessel 1. By spraying water in this manner, a proper water content can be maintained.

The agitation scraper 3 agitates a content in the reaction vessel 1. There are no restrictions to the number or the size of the agitation scrapers 3, and they can be appropriately chosen such that the content in the reaction vessel 1 is adequately mixed and agitated.

Although the shaft 4 can be manually or electrically rotated, it is preferably rotated using a motor 7 as shown in FIG. 1. There are no particular restrictions to a rotation speed of the shaft 4 as long as with the rotation speed, a microorganism-supporting carrier, a biological material and water in the reaction vessel 1 are adequately mixed and agitated.

The pipe for removing an aqueous solution 5 is a pipe removing water in the reaction vessel 1, that is, an aqueous solution containing a decomposition product from the reaction vessel 1. Specifically, an aqueous solution containing a decomposition product is preferably filtered through a screen plate 6 with an aperture size of 1 to 2 mm placed under the reaction vessel and then removed through the pipe for removing an aqueous solution 5, as shown in FIG. 1.

In a preferable embodiment of decomposing a biological material using this decomposition apparatus, 20 to 500 parts by weight of a biological material and 5 to 70 parts by weight of water are added to 100 parts by weight of an aqueous polyvinyl alcohol carrier in a reaction vessel 1, and the mixture is mixed and agitated. There are no particular restrictions to the order of addition to the reaction vessel 1; for example, the biological material is added to the reaction vessel 1 before supplying water through the water spray pipe 2, water is supplied through the water spray pipe 2 before adding a biological material to the reaction vessel 1, or water is supplied through the water spray pipe 2 while a biological material is added to the reaction vessel 1. Furthermore, it is preferable to supply water through the water spray pipe 2 such that the amount of water in the reaction vessel 1 is 5 to 70 parts by weight based on 100 parts by weight of a microorganism-supporting carrier.

Then, to the reaction vessel 1, a biological material is added and water is supplied through the water spray pipe 2, and the content is mixed and agitated with the agitation scraper 3, to initiate decomposition of the biological material. Furthermore, water supplied through the water spray pipe 2 washes the content in the reaction vessel 1 for extracting a decomposition product to give an aqueous solution, which is then removed through the pipe for removing an aqueous solution 5 from the decomposition apparatus. Thus, a solution containing the decomposition product is obtained in an about 20-fold weight based on the weight of the biomass material loaded.

EXAMPLES

There will be specifically described the present invention with reference to, but not limited to, Examples. In the description below, an aqueous solution containing a decomposition product is sometimes referred to as "BDS (Biomass Digested Solution)".

In the examples below, an aqueous polyvinyl alcohol carrier (hereinafter, referred to as "carrier A"), a polypropylene carrier (hereinafter, referred to as "carrier B"), and a low hydrous polyvinyl alcohol carrier (hereinafter, referred to as "carrier C") are used as a carrier. Carrier A is a spherical polyvinyl alcohol-based hydrous gel having a porous structure produced by adding dropwise an aqueous solution of polyvinyl alcohol and an alginate to an aqueous solution containing an aqueous solution of calcium chloride followed by formalization with formalin. This carrier A has a sphere-equivalent diameter of 4 mm and a water content of 89 % by weight. Carrier B is a non-porous cube (about 5 mm on a side) with a water content of 0 % by weight. Carrier C is a spherical polyvinyl alcohol type hydrous gel having a porous structure produced as described for carrier A at a higher formalization temperature. Carrier C has a sphere-equivalent diameter of 4 mm and a water content of 45 % by weight.

[Preparation of a microorganism-supporting carrier]

Bacillus bacteria were inoculated into a liquid medium (glucose 5 %, peptone 0.5 %, yeast extract 0. 3 %,pH 6. 8) and cultured at 28 °C for 48 hours. To the Bacillus culture medium thus prepared was added carrier A, carrier B or carrier C in a 1.5-fold amount as a wet weight, and the medium was adequately agitated and then allowed to stand at room temperature (22 °C) to prepare a bacterium-supporting carrier.

[Measurement of the number of bacteria supported]

One mL of a carrier supporting microorganisms (carrier A, B or C) was pulverized and suspended in sterilized water. Bacteria supported on each carrier were cultured on a nutrient agar plating medium and the number of bacteria was measured after 72 hours and 168 hours. The results are shown in Table 1. Hereinafter, carrier A, B or C supporting microorganism is referred to as a microorganism-supporting carrier A, B or C, respectively.

[Table 1]

| Carrier | Number of microorganisms supported (hundred million/mL) | |
| --- | --- | --- |
| | 72 hours | 168 hours |
| Microorganism-supporting carrier A | 22 | 120 |
| Microorganism-supporting carrier B | 4 | 8 |
| Microorganism-supporting carrier C | 13 | 58 |

[Measurement of a biological material decomposition rate (liquefaction degree)]

For determining a decomposition rate of a biological material by Bacillus bacteria, the total solubilized saccharide amount in an aqueous solution containing a decomposition product was measured. Specifically, an aqueous solution containing a decomposition product was centrifuged, and the supernatant was removed and measured for the total solubilized saccharide amount. Herein, the total solubilized saccharide amount is a collective designation of saccharides which are soluble in water. When a biomass is a plant, substances constituting the plant are present in structures surrounded by biological surface materials made from water-insoluble polysaccharides such as cellulose and hemicellulose. When these biological surface coats are decomposed to be water-soluble, biological ingredients give an aqueous solution. Therefore, the amount of water-soluble saccharides (total solubilized saccharides) can be measured as an indicator of a degree of plant decomposition, which is a parameter for determining a biomass decomposition rate. The total solubilized saccharide amount was measured by a phenol-sulfuric acid method as described in "Shokuhin Bunseki No Jissai (Practice of Food Analysis) (Takaaki Manabe, Saiwai Shobo, 2003).

[Demonstration of effects of biological material decomposition using a microorganism-supporting carrier]

Example 1

In 500 mL vessel was placed 25 g of turfgrass, to which were added 25 mL of microorganism-supporting carrier A (25.8 g, total supported bacterium number: 55 billion) and 50 parts by weight of water based on 100 parts by weight of the microorganism-supporting carrier A, and the mixture was stirred at 28 °C for 72 hours. Subsequently, the aqueous solution containing a decomposition product was taken out of the vessel and measured for the total solubilized saccharized amount. The results are shown in Table 2.

Comparable Example 1

The process was conducted as described in Example 1, substituting Bacillus bacteria (bacterium number: 55 billion) for microorganism-supporting carrier A. The results are shown in Table 2.

Comparative Example 2

The process was conducted as described in Example 1, substituting carrier A for microorganism-supporting carrier A and using Bacillus bacteria (bacterium number : 55 billion) The results are shown in Table 2.

Comparative Example 3

The process was conducted as described in Example 1, substituting microorganism-supporting carrier B (total supported microorganism number: 20 billion) for microorganism-supporting carrier A. The results are shown in Table 2.

Comparative Example 4

The process was conducted as described in Example 1, except that microorganism-supporting carrier A was not added to the vessel. The results are shown in Table 2.

[Table 2]

| | | Example 1 | Comparative Example 1 | Comparative Example 2 | Comparative Example 3 | Comparative Example 4 |
|---|---|---|---|---|---|---|
| Carrier | Microorganism-supporting carrier A (mL) | 25 | | | | |
| | Microorganism-supporting carrier B (mL) | | | | 25 | |
| | Carrier A (mL) | | | 25 | | |
| Bacillus bacteria | | | ○ | ○ | | |
| Biomass | Turfgrass (g) | 25 | 25 | 25 | 25 | 25 |
| Water content (parts by weight / 100 parts by weight of a carrier) | | 50 | 50 | 50 | 50 | 50 |
| Total solubilized saccharide amount (mg/50 mL) | | 165 | 15 | 3 | 2> | 2> |

Example 2

In a 500 mL vessel was placed 25 g of a red-cabbage pigment extraction residue (a residue after extraction of red-cabbage pigment), to which were added 25 mL of microorganism-supporting carrier A (25.8 g, total supported microorganism number: 55 billion) and 50 parts by weight of water based on 100 parts by weight of the microorganism-supporting carrier A, and the mixture was agitated at 28 °C for 72 hours. Then, an aqueous solution containing a decomposition product was taken out of a vessel and measured for the total solubilized saccharide amount. The results are shown in Table 3.

Comparable Example 5

The process was conducted as described in Example 2, substituting Bacillus bacteria (bacterium number: 55 billion) for microorganism-supporting carrier A. The results are shown in Table 3.

Comparative Example 6

The process was conducted as described in Example 2, substituting carrier A for microorganism-supporting carrier A and using Bacillus bacteria (bacterium number : 55 billion). The results are shown in Table 3.

Comparative Example 7

The process was conducted as described in Example 2, substituting microorganism-supporting carrier B (total supported microorganism number: 20 billion) for microorganism-supporting carrier A. The results are shown in Table 3.

Comparative Example 8

The process was conducted as described in Example 2 except that microorganism-supporting carrier A was not added to the vessel. The results are shown in Table 3.

[Table 3]

| | | Example 2 | Comparative Example 5 | Comparative Example 6 | Comparative Example 7 | Comparative Example 8 |
|---|---|---|---|---|---|---|
| Carrier | Microorganism-supporting carrier A (mL) | 25 | | | | |
| | Microorganism-supporting carrier B (mL) | | | | 25 | |
| | Carrier A (mL) | | | 25 | | |
| Bacillus bacteria | | | ○ | ○ | | |
| Biomass | Red cabbage (g) | 25 | 25 | 25 | 25 | 25 |
| Water content (parts by weight / 100 parts by weight of a carrier) | | 50 | 50 | 50 | 50 | 50 |
| Total solubilized saccharide amount (mg/50 mL) | | 1403 | 132 | 28 | 20 | 2> |

Examples 3 and 4, and Comparative Examples 9 and 10

In a decomposition apparatus (vessel volume: 3 L) was placed 2 kg of turfgrass, to which were added 1 L of microorganism-supporting carrier A (total supported microorganism number: 2,200 billion), 1 L of microorganism-supporting carrier B (total supported microorganism number: 400 billion) and water in the amount described in Table 4 based on 100 parts by weight of microorganism-supporting carrier A, and the mixture was agitated at 28 °C for 36 hours. Twenty four hours after initiation of agitation, an aqueous solution containing a decomposition apparatus was taken out of the vessel. Then, the aqueous solution was centrifuged and the supernatant was removed and measured for the total solubilized saccharide amount. The results are shown in Table 4.

[Table 4]

| | | Example 3 | Example 4 | Comparative Example 9 | Comparative Example 10 |
|---|---|---|---|---|---|
| Carrier | Microorganism-supporting carrier A (L) | 1 | 1 | 1 | 1 |
| | Microorganism-supporting carrier B (L) | 1 | 1 | 1 | 1 |
| Biomass | Turfgrass (kg) | 2 | 2 | 2 | 2 |
| Water content (parts by weight / 100 parts by weight of a carrier) | | 50 | 10 | 100 | 300 |
| Total solubilized saccharide amount (mg/50 mL) | | 1200 | 550 | 12 | 5> |

The results in Table 4 show that a water content during apparatus operation influences biomass decomposition.

[Demonstration of utility as a fertilizer]

Example 5

In order to verify practical utility of a BDS, its effects on germination of Japanese mustard spinach seeds was studied by the following process.

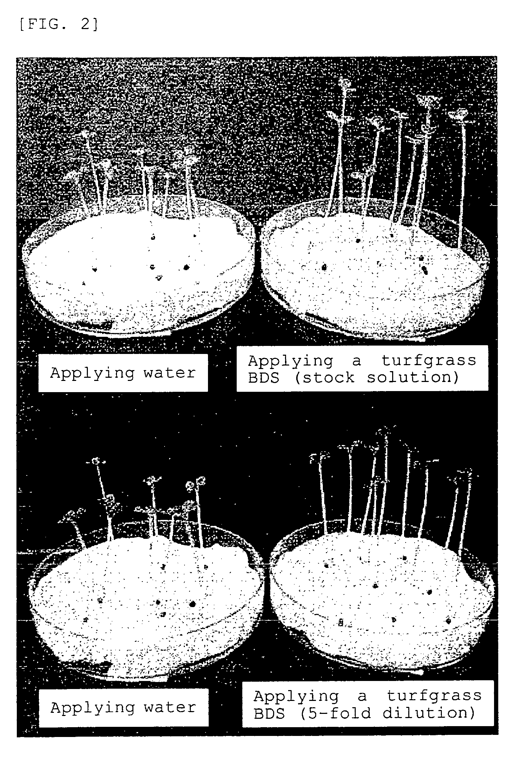

Ten milliliters of the BDS obtained in Example 3 was diluted with distilled water in the proportion shown in Table 5. Then, seeds of Japanese mustard spinach (Tohoku Seed Co., Ltd.) were sown on an absorbent cotton bedded in a petri dish and then 10 mL of a solution described in Table 5 was absorbed by the cotton. Then, the seeds of Japanese mustard spinach were cultured for germination at room temperature (average temperature: 23 °C). The results are shown in FIG. 2 and Table 5.

[Table 5]

| Solution added (10mL) | Average stem length (mm) |
| --- | --- |
| Distilled water | 39 |
| Turfgrass BDS stock solution | 51 |
| Turfgrass BDS 5-fold diluted solution | 50 |
| Turfgrass BDS 10-fold diluted solution | 47 |
| Turfgrass BDS 25-fold diluted solution | 44 |
| Turfgrass BDS 50-fold diluted solution | 41 |

The results show that the BDS in Example 3 is effective for accelerating Japanese mustard spinach, depending on a concentration of the BDS. It was observed that the BDS stock solution was about 1.3 times as effective as that without the solution, leading to yield increase by 30 %. Thus, it was shown that the BDS in Example 3 was effective as a fertilizer for seed germination.

Example 6

In order to verify practical utility of a BDS, its effects on growth of Japanese mustard spinach after seed germination was studied as described below.

A gardening planter was filled with Kanuma soil to a depth of about 10 cm, over which was layered a field soil containing 15 g of magnesium lime to a thickness of 15 cm. Two such planters were prepared. In one planter (planter A), the BDS obtained in Example 3 was sprayed at a rate of 2 liters per 1 m$^2$. In the other planter (planter B), water was sprayed at a rate of 2 liters per 1 m$^2$. These planters were allowed to stand for 5 days and subjected spraying as described above. After additional 2 days, seeds of Japanese mustard spinach (Tohoku Seed Co., Ltd.) were sown.

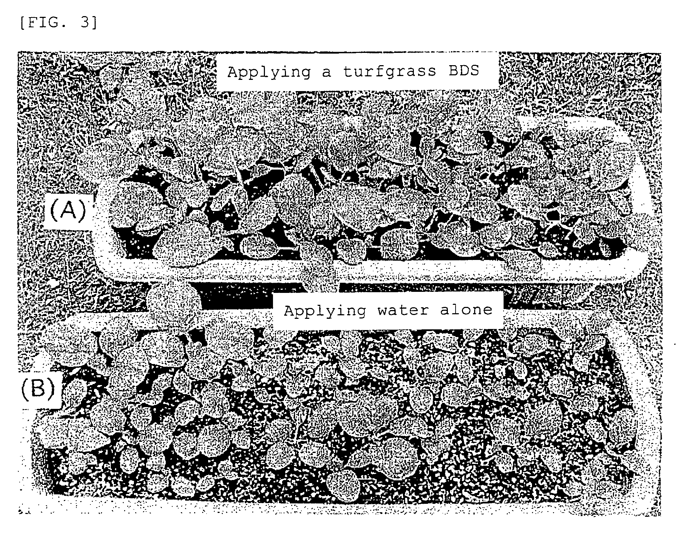

Subsequently, at intervals of 4 to 5 days, the BDS in Example 3 and water were sprayed to planters A and B, respectively, at a rate of 2 liters per 1 m$^2$. After germination, Japanese mustard spinach was weighed in grams for planters A and B at day 20, and an average of 10 heads was recorded as a weight of Japanese mustard spinach for each planter. As a result, a weight of Japanese mustard spinach was 29.5 g for planter A while being 18.9 g for planter B. The results are shown in FIG. 3.

The results indicate that, with spraying the BDS in Example 3, the plant grew twice as much as with spraying water alone, demonstrating that the BDS in Example 3 is effective as a fertilizer for growing of Japanese mustard spinach.

Example 7

In order to verify practical utility of a decomposition method according to the present invention, a garbage was decomposed by the decomposition method.

A general garbage is a biomass mixture derived from different sources such as vegetables, meats, cereals and processed foods. In this example, a biomass mixture having a composition shown in Table 6 was prepared, taking a general garbage composition into consideration.

[Table 6]

| Element | % by weight |
|---|---|
| Vegetables | 40 |
| Cereals (heated) | 30 |
| Meats | 15 |
| Processed noodles | 5 |
| Others | 10 |

The above biomass mixture (50 kg) and microorganism-supporting carrier A (20 L) were loaded in a reaction vessel, to which was supplied 40 parts by weight of water based on 100 parts by weight of the microorganism-supporting carrier A at 25 °C at intervals of 30 min for decomposition, and a BDS was removed. A composition of the BDS obtained after decomposition for 72 hours was analyzed, and the results are shown in Table 7.

[Table 7]

| Analysis item | Value |
|---|---|
| Hydrogen ion concentration (pH) (22 °C) | 6.3 |
| Potassium (mg/L) | 4 |
| Total phosphate (mg/L) | 1.1 |
| Total nitrogen (mg/L) | 3.9 |
| BOD (mg/L) | 90 |
| COD (Mn) (mg/L) | 25 |
| Suspended substance amount (SS) (mg/L) | 34 |

The above results show that a decomposition product produced by decomposition of a garbage has a composition suitable as a fertilizer.

From the results described above, liquefaction of a solid biomass resource, which has been difficult by the prior art, can be easily achieved, allowing elements in the biomass to be dissolved and extracted. Thus, biomass elements can be easily utilized. Since biomass elements include those which can be a source for a fertilizer and can be easily utilized, the present invention is significantly valuable.

DESCRIPTION OF SYMBOLS

1:     Reaction vessel
2:     Water spray pipe
3:     Agitation scraper
4:     Shaft
5:     Pipe for removing an aqueous solution
6:     Screen plate
7:     Motor
8:     Apparatus casing
9:     Water inlet pipe
10:    Collection tray

**Claims**

1. A method for decomposing a biological material by microorganisms supported on a carrier, **characterized in that** the carrier is an aqueous polyvinyl alcohol carrier and 20 to 500 parts by weight of the biological material and 5 to 70 parts by weight of water based on 100 parts by weight of the aqueous polyvinyl alcohol carrier are mixed and agitated in a reaction vessel.

2. The method for decomposing a biological material as claimed in Claim 1, wherein an aqueous solution containing a decomposition product is taken out of the reaction vessel.

3. The method for decomposing a biological material as claimed in Claim 2, wherein an aqueous solution containing a decomposition product is continuously taken out of the reaction vessel while water is continuously or intermittently supplied to the reaction vessel.

4. The method for decomposing a biological material as claimed in any of Claims 1 to 3, wherein the microorganisms are bacteria of Bacillus genus.

5. The method for decomposing a biological material as claimed in any of Claims 1 to 4, wherein the biological material is at least one selected from the group consisting of an agricultural waste, a livestock waste, a fishery waste, a food processing waste and a kitchen waste.

6. The method for decomposing a biological material as claimed in any of Claims 1 to 5, wherein 20 to 300 parts by weight of a hard carrier based on 100 parts by weight of the aqueous polyvinyl alcohol carrier is further added to the reaction vessel and the mixture is then mixed and agitated.

7. A method for producing a liquid fertilizer by decomposing a biological material by microorganisms supported on a carrier, **characterized in that** the carrier is an aqueous polyvinyl alcohol carrier, and 20 to 500 parts by weight of the biological material and 5 to 70 parts by weight of water based on 100 parts by weight of the aqueous polyvinyl alcohol carrier are mixed and agitated, and an aqueous solution containing a decomposition product is taken out of the reaction vessel.

8. An apparatus for decomposing a biological material by microorganisms supported on a carrier, comprising a reaction vessel which contains an aqueous polyvinyl alcohol carrier supporting microorganisms; means for supplying water to the reaction vessel; means for agitating a biological material in the reaction vessel; and means for taking an aqueous solution containing a decomposition product out of the reaction vessel.

[FIG. 1]

[FIG. 2]

Applying water

Applying a turfgrass BDS (stock solution)

Applying water

Applying a turfgrass BDS (5-fold dilution)

[FIG. 3]

(A) Applying a turfgrass BDS

(B) Applying water alone

## INTERNATIONAL SEARCH REPORT

| International application No. |
| --- |
| PCT/JP2012/075791 |

A.   CLASSIFICATION OF SUBJECT MATTER
*B09B3/00*(2006.01)i, *C05F1/00*(2006.01)i, *C05F3/00*(2006.01)i, *C05F5/00*
(2006.01)i, *C05F9/00*(2006.01)i, *C05G5/00*(2006.01)i, *C12M1/00*(2006.01)i,
*C12N1/00*(2006.01)i, *C12N1/20*(2006.01)i, *C12N11/08*(2006.01)n
According to International Patent Classification (IPC) or to both national classification and IPC

B.   FIELDS SEARCHED

Minimum documentation searched (classification system followed by classification symbols)
B09B1/00-5/00, C02F11/00-11/20, C05F1/00-17/00, C05G5/00, C12M1/00-1/42,
C12N1/00-1/38

Documentation searched other than minimum documentation to the extent that such documents are included in the fields searched
    Jitsuyo Shinan Koho       1922–1996   Jitsuyo Shinan Toroku Koho   1996–2012
    Kokai Jitsuyo Shinan Koho   1971–2012   Toroku Jitsuyo Shinan Koho   1994–2012

Electronic data base consulted during the international search (name of data base and, where practicable, search terms used)
   WPI

C.   DOCUMENTS CONSIDERED TO BE RELEVANT

| Category* | Citation of document, with indication, where appropriate, of the relevant passages | Relevant to claim No. |
| --- | --- | --- |
| X<br>Y | JP 11-290825 A   (Kanebo, Ltd.),<br>26 October 1999 (26.10.1999),<br>claims; paragraphs [0001], [0030], [0038],<br>[0039], [0047]; examples<br>(Family: none) | 1,4-6<br>2,3,7,8 |
| X<br>Y | JP 10-43728 A   (Mitsubishi Materials Corp.),<br>17 February 1998 (17.02.1998),<br>claims; paragraphs [0001], [0007], [0008];<br>examples<br>(Family: none) | 1,4-6<br>2,3,7,8 |
| X<br>Y | JP 2001-25748 A   (Rengo Co., Ltd.),<br>30 January 2001 (30.01.2001),<br>claims; paragraphs [0001], [0017], [0020],<br>[0021]; examples<br>(Family: none) | 1,4-6<br>2,3,7,8 |

☒   Further documents are listed in the continuation of Box C.      ☐   See patent family annex.

| | |
| --- | --- |
| *   Special categories of cited documents:<br>"A"  document defining the general state of the art which is not considered<br>     to be of particular relevance<br>"E"  earlier application or patent but published on or after the international<br>     filing date<br>"L"  document which may throw doubts on priority claim(s) or which is<br>     cited to establish the publication date of another citation or other<br>     special reason (as specified)<br>"O"  document referring to an oral disclosure, use, exhibition or other means<br>"P"  document published prior to the international filing date but later than<br>     the priority date claimed | "T"  later document published after the international filing date or priority<br>     date and not in conflict with the application but cited to understand<br>     the principle or theory underlying the invention<br>"X"  document of particular relevance; the claimed invention cannot be<br>     considered novel or cannot be considered to involve an inventive<br>     step when the document is taken alone<br>"Y"  document of particular relevance; the claimed invention cannot be<br>     considered to involve an inventive step when the document is<br>     combined with one or more other such documents, such combination<br>     being obvious to a person skilled in the art<br>"&"  document member of the same patent family |

| Date of the actual completion of the international search<br>   22 October, 2012 (22.10.12) | Date of mailing of the international search report<br>   30 October, 2012 (30.10.12) |
| --- | --- |
| Name and mailing address of the ISA/<br>   Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2012/075791

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JP 8-309318 A  (Matsushita Electric Industrial Co., Ltd.),<br>26 November 1996 (26.11.1996),<br>claims; paragraphs [0001], [0023]<br>(Family: none) | 1,4-6<br>2,3,7,8 |
| Y | JP 2001-314836 A  (Ohama Kiko Kabushiki Kaisha),<br>13 November 2001 (13.11.2001),<br>claims; paragraphs [0001], [0019]; fig. 1, 2<br>(Family: none) | 2,3,7,8 |
| P,X | JP 2012-96957 A  (Takuo SAKAI),<br>24 May 2012 (24.05.2012),<br>claims; paragraphs [0016], [0017]; examples<br>(Family: none) | 1-8 |

Form PCT/ISA/210 (continuation of second sheet) (July 2009)

**REFERENCES CITED IN THE DESCRIPTION**

**Patent documents cited in the description**

- JP 8112582 A


**Non-patent literature cited in the description**

- *Shokuhin Bunseki No Jissai,* 2003